(19)

Europäisches
Patentamt
European
Patent Office
Office européen
des brevets

(11) **EP 2 422 787 A1**

(12) **EUROPEAN PATENT APPLICATION**

(43) Date of publication:
**29.02.2012 Bulletin 2012/09**

(21) Application number: **10382233.4**

(22) Date of filing: **17.08.2010**

(51) Int Cl.:
**A61K 31/549** (2006.01)　　　**A61P 25/14** (2006.01)
**A61P 25/28** (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Neurotec Pharma, S.L.**
**08028 Barcelona (ES)**

(72) Inventors:
• **Pugliese, Marco**
  **E-08810, Sant Pere de Ribes - Barcelona (ES)**
• **Espinosa Parrilla, Juan Francisco**
  **E-08915, Badalona (Barcelona) (ES)**

• **Virgili Treserres, Noemí**
  **E-43883, Roda de Barà (Tarragona) (ES)**
• **Mancera Aroca, Pilar**
  **E-43883, Roda de Barà (Tarragona) (ES)**
• **Pastén Zamorano, Andrea**
  **E-08026, Barcelona (ES)**
• **Mahy Gehenne, Josette-Nicole**
  **E-07460, Pollença (Mallorca) (ES)**
• **Rodríguez Alluè, Manuel**
  **E-08980, Sant Feliu de Llobregat - Barce (ES)**

(74) Representative: **ABG Patentes, S.L.**
**Avenida de Burgos 16D**
**Edificio Euromor**
**28036 Madrid (ES)**

(54) **Diazoxide for use in the treatment of amyotrophic lateral sclerosis (als)**

(57) The invention relates to the diazoxide or a pharmaceutically acceptable salt thereof for use as a medicament at low doses to treat amyotrophic lateral sclerosis.

EP 2 422 787 A1

**Description**

**FIELD OF THE INVENTION**

The invention relates to diazoxide or a pharmaceutically acceptable salt thereof for use as a medicament at low doses to treat amyotrophic lateral sclerosis (ALS) and to compositions comprising low doses of diazoxide for use in the treatment of a mammal afflicted with ALS.

**BACKGROUND OF THE INVENTION**

Amyotrophic lateral sclerosis (ALS) is a term used to cover the spectrum of neurodegenerative syndromes characterized by progressive muscular paralysis reflecting degeneration of motor neurons in the brain and spinal cord. However, the term used in modem clinical practice to indicate the commonest form of the disease is Classical (Charcot's) ALS and now is more familiarly known in the United States as Lou Gehrig's disease. In Europe, Australia, New Zealand and Asia the term more commonly used is motor neuron disease (MND). Other syndromes related to this spectrum of disorders include progressive bulbar palsy (PBP), progressive muscular atrophy (PMA), primary lateral sclerosis (PLS) and ALS with multi-system involvement (Wijesekera LC and Leigh PN. Amyotrophic lateral sclerosis. Orphanet Journal of Rare Disease 2009, 4:3).

ALS is one of the most common neurodegenerative disorders, with an incidence of 1 to 2 per 100,000 and a prevalence of 4 to 6 per 100,000; as many as 30,000 Americans have the disease at any given time (Worms PM. The epidemiology of motor neuron disease: a review of recent studies. J Neurol Sci 2001, 191:3-9). The incidence in males is higher than in females (1.6:1). 5-10% of patients have a positive family history of ALS, most commonly with an autosomal dominant inheritance pattern. ALS is a disease of mature adults, with a median age of onset of 55 years and its frequency increases with age until age 75. Overall 50% of patients die within the first three years since the first clinical manifestations. Apart from age or a positive family history, a number of factors and environmental toxins have been further studied as risk factors. A high relative risk was described in smokers, soccer players, especially Italians and veterans of the Gulf War.

Sporadic and familial ALS (SALS and FALS, respectively) are clinically and pathologically similar, suggesting a common pathogenesis. Both forms produce similar pathological hallmarks, including progressive muscle weakness, atrophy, and spasticity, each of which reflects the degeneration and death of upper and lower motor neurons. Denervation of the respiratory muscles and diaphragm is generally the fatal event. Actually, in current medical practice, the terms "bulbar onset ALS" and "spinal onset ALS" have replaced the terms PBP and Charchot's ALS. Approximately two thirds of patients with typical ALS have a spinal form of the disease (limb onset) and present symptoms related to focal muscle weakness and wasting, where the symptoms may start either distally or proximally in the upper and lower limbs. Gradually, spasticity may develop in the weakened atrophic limbs, affecting manual dexterity and gait. Patients with bulbar onset ALS usually present with dysarthria and dysphagia for solid or liquids, and limbs symptoms can develop almost simultaneously with bulbar symptoms, and in the vast majority of cases will occur within 1-2 years (Mitchell JD and Borasio GD. Amyotrophic lateral sclerosis. Lancet 2007, 369:2031-41). Paralysis is progressive and leads to death due to respiratory failure within 2-3 years for bulbar onset cases and 3-5 years for limb onset ALS cases.

Approximately 10% of cases of ALS are familial. The remaining 90% are sporadic and though to be multifactorial, with both environmental and genetic components contributing to disease susceptibility. The genetics of both FALS and SALS is complex. 20% of cases with autosomal dominant FALS and 2% of patients with SALS show mutations in the copper/zinc superoxide dismutase (SOD1) gene on chromosome 21. Mutations in the gene are though to cause disease through a toxic gain of function rather than causing impairment of the antioxidant function of the SOD1 enzyme. Although genes different from other than SOD1 have been associated with familial ALS, including alsin (ALS2), senataxin (ALS4) or Angiogenin, the genetic defect remains to be identified in the majority of cases (Pasinelli P and Brown RH. Molecular biology of amyotrophic lateral sclerosis: insight from genetics. Nat Rev Neurosci 2006, 7:710-723).

ALS is a multifactorial disease with a complex interplay between multiple pathogenic cellular mechanisms including genetic factors, protein misfolding and aggregation, oxidative damage and mitochondrial dysfunction, cytoskeletal abnormalities and defective axonal transport. Motor neuron damage is enhanced by noxious signals originating from non-neuronal neighboring cells (astro- and microglial cells), where mutant SOD1 induces excitotoxicity, inadequate growth factor signaling, and an inflammatory response that accelerates disease progression (Cozzolino M et al. Amyotrophic Lateral Sclerosis: From current developments in the laboratory to clinical implications. Antiox Redox Sign 2008, 10:406-43). Increasing evidence indicates that cellular functions impaired as a consequence of the expression of mutant SOD1 converge on pathways that could be activated in sporadic ALS by other toxic factors, and it is hoped that therapies effective in mutant SOD1 animal models will translate to sporadic and non- SOD1-linked ALS.

Several studies revealed that inflammatory reactions are highly prominent in ALS patients. In these patients, microglial activation, not present in healthy controls, is a major component of the CNS pathology and increased with disease severity. In response to signals originating from damaged motor neurons and the surrounding astrocytes, microglial cells downregulate the production of neurotrophic factors which sustain neurons in resting condition, and begin to produce proinflammatory cytokines. As long as microglia activation proceeds, inflammatory cytokines accumulate, together with reactive oxygen (ROS) and nitrogen (RNS) species, and glutamate. The resulting inflammatory environment contributes to the degeneration of motor neurons and to the progression of the disease (Turner MR et al. Evidence of widespread cerebral microglial activation in amyotrophic lateral sclerosis: an [11C](R)-PK11195 positron emission tomography study. 2004, Neurobiol Dis 15:601-609).

A major breakthrough in the ALS research was the discovery that - 20% FALS cases were due to mutant SOD1. In 1994, transgenic animals were developed which carried mutations in the gene for the human Cu/Zn SOD that were shown to be responsible for human ALS (Gurney ME et al. Motor neuron degeneration in mice that express a human Cu/Zn superoxide dismutase mutation. 1994, Science 264:1772-5). The initial mutations produced the substitution of glycine for alanine at position 93 (G93A), and of alanine for valine at position 4 (A4V). Other SOD1 mutations have been expressed in transgenic mice. The commonest, in addition to G93A and A4V, are glutamate for arginine substitution at positions 37 (G37R) and 85 (G85R). These animals developed progressive anterior horn cell disease with hindlimb weakness, impaired leg extension and shortened stride length, and continues to complete paralysis of the limbs, principally the rear ones, within few days and died from respiratory failure.

Currently, the transgenic SOD1G93A mouse model remains the best established model for ALS/MND for the preclinical evaluation of potentially disease-modifying drugs (Ludolph AC et al. Guidelines for the preclinical in vivo evaluation of pharmacological active drugs for ALS/MND: Report on the 142nd ENMC international workshop. 2007, Amyothr Lat Sclerosis 8:217-223). In this model cellular alterations are mainly characterized by vacuolar degeneration of motor neurons and their processes at the early stages, followed by neuronal loss and atrophy of the ventral horns in the spinal cord at the late stages. Since no symptomatic or pathological evident differences exist between FALS and SALS, it has been assumed that the mechanisms underlying both types of the disease are shared. Therefore, transgenic SOD1 animals have been extensively used in numerous studies related to ALS, principally because the phenotype they display is elicited by the only proven cause of the disease. This model has also been the golden standard for drugs and therapies testing at experimental stages.

There is no cure for ALS. At present, there is no treatment that can influence significantly the course of the disease, but only palliative therapies that aim only to improve symptoms. Many putative disease-modifying strategies for ALS have been tested in clinical trials but only one drug, riluzole (Rilutek, Sanofi-Aventis) has so far been approved by Food and Drug Administration (FDA) and European Medicines Agency (EMEA). The mechanism of action of riluzole is not entirely certain but is though to include inhibition of glutamate release from pre-synaptic terminals and increasing of extracellular glutamate uptake. However, the initial trials showed a very modest (mean three months) increase in survival. More than 10 placebo controlled trials have been performed since the introduction of riluzole, but have failed to show an additional beneficial effect on disease course. The drug is generally tolerated with the most common side effects being asthenia and nausea. Recently lithium has been shown to slow down the progression of ALS quite dramatically. Large-scale trials of lithium in ALS are presently underway. Other current multicentre clinical trials include, among others: Olesoxime (TRO19622), Arimoclomol, AVP-923, Memantine, Talampanel, Olanzapine, Ceftriaxone, ONO-2506PO, antioxidants (such as coenzyme Q10, Vitamin E), Pyrimethamine Minocycline and tamoxifen (Kollewe K et al. Amyotrophic lateral sclerosis: Current clinical trials and underlying pathomechanisms. 2008, Nervenartz. 79: 653-61).

The mainstay of ALS care remains, therefore, symptomatic and all efforts should be made to improve quality of life and help to maintain the patient's autonomy for as long as possible. The main symptoms encountered in ALS and their medical managements are shown in table I.

**Table I. Suggestions for symptomatic treatments in ALS**

| Symptom | Suggested Drugs |
|---|---|
| Cramps and fasciculation | |
| Mild | Magnesium<br>Vitamin E |
| Severe | Carbamazepine<br>Phenytoin<br>Quinine sulphate<br>Gabapentin |
| | |

(continued)

| Symptom | Suggested Drugs |
|---|---|
| Spasticity | Baclofen<br>Tizanidine<br>Dantrolene<br>Memantine<br>Tetrazepam<br>Botulinum toxin type A |
| Sialorrhea | Amitriptyline<br>Atropine sulphate<br>Hyoscine hydrobromide<br>Hyoscine butylbromide<br>Hyoscine scopoderm<br>Glycopirrolate |
| Persistent saliva and bronchial secretions | Carbocisteine<br>Propanolol<br>Metoprolol |
| Excessive or violent yawning | Baclofen |
| Laryngospasm | Lorazepam |
| Pain | Analgesic<br>Non-steroidal anti-inflammatory drugs<br>Opioids |
| Depression | Antidepressants |
| Anxiety | Lorazepam |
| Pathological laughing or crying | Tryciclic antidepressant<br>Selective serotonin-reuptake inhibitors<br>Levodopa |
| Fatigue | Modanafil |
| Sleep disturbances | Zolpidem<br>Amitriptyline |
| Urinary urgency | Oxybutynin |
| Constipation | Lactulose |

In summary, it is highly desirable to provide new therapeutic agents for the treatment of ALS applicable to all clinical presentations of the disease, easily to administer and with no side effects in their acute and long-term therapeutic use.

It has been shown that activated microglia, one of the elements involved in ALS inflammation, expresses $K_{ATP}$ channels (Ramonet D et al. Putative glucosensing property in rat and human activated microglia. Neurobiol Dis 2004, 17:1-9) and it has been suggested that this may represent a new therapeutic target.

WO2006/000607 Al discloses the potential use of $K_{ATP}$ channel openers (KCO) for the treatment of CNS chronic inflammation associated to a disease in mammals. The application also disclosed that suitable doses were to be determined by a doctor or veterinary but would lie within the range of 0.01 to 1000 mg/kg/day.

Diazoxide is a benzothiadiazine that acts as a $K_{ATP}$ channel opener (Mannhold R. KATP channel openers: structure-activity relationships and therapeutic potential. Med Res Rev 2004, 24:213-66) and that has been used until now against human hypertension and hypoglycemia. Diazoxide is administered parenterally in the form of minibolus of 1-3 mg/kg (37-111 mg/m$^2$) up to a maximum of 150 mg (185 mg/m$^2$) in a single injection every 5 to 15 minutes to treat hypertensive emergencies (Hyperstat®) and it is also used orally to manage refractory malignant hypertension in a dose range of 600-800 mg per day (370-493 mg/m$^2$/day) (Fang P, MacDonald I, Laver M, Hua A, Kincaid-Smith P. Oral diazoxide in uncontrolled malignant hypertension. Med J Aust. 1974 Oct 26;2(17):621-4). Effective oral doses in adult humans for the treatment of hypoglycemia are 3-8 mg/kg/day (111-296 mg/m$^2$/day) (Proglycem®). However, these doses frequently cause severe side effects such as oedema and hirsutism.

Until now no studies have investigated the effectiveness of diazoxide in ALS. In addition, research related to the dose-response effect of diazoxide in different brain damage situations has shown that high doses are required to get some beneficial effect. Gantenbein et al. showed that 100 mg/kg (300 mg/m$^2$) of diazoxide are required to reduce the bupivacaine-induced acute CNS toxicity in mice by increasing the period of latency of the convulsions caused by bupivacaine (Gantenbein M et al. 1995. Life Sciences, 57: 113-116). Farkas E et al. showed that 5 mg/day (143 mg/m$^2$/day) diazoxide in rats and dimethyl sulphoxide prevents cerebral hypoperfusion-related learning dysfunction and brain damage after carotid artery occlusion (Farkas E et al. Brain Research. 2004, 1008:252-260). Lenzser G et al. showed that diazoxide preconditioning is capable of attenuating global cerebral ischemia-induced blood-brain barrier permeability when administered to rats at 20 mg/kg/day (120 mg/m$^2$/day) but shows no effect at 6 mg/kg/day (36 mg/m$^2$/day) (Lenzser G et al. Brain Research. 2005, 1051:72-80).

Notwithstanding the above, it would be of great interest to develop a method of treatment of amyotrophic lateral sclerosis (ALS) which is devoid of undesirable side effects associated with the use of KCOs such as hyperglycemia.

## SUMMARY OF THE INVENTION

Inventors have surprisingly found that daily doses of diazoxide well below those previously used in the treatment of other therapeutic conditions treatable with diazoxide result in an unexpected and advantageous effect by improving clinical manifestations of amyotrophic lateral sclerosis (ALS) and survival rate without causing undesired side effects, including therapeutic effects which are undesirable in patients affected only with ALS such as hyperglycemia, normally present when diazoxide is administered at doses used until now in therapy.

In one aspect, the invention relates to diazoxide or a pharmaceutically acceptable salt thereof for use as a medicament at a daily dose of from 0.15 mg/m$^2$/day to 13.00 mg/m$^2$/day expressed as mg/m$^2$/day of diazoxide free base in the treatment of a mammal afflicted with amyotrophic lateral sclerosis (ALS).

In another aspect, the invention relates to a pharmaceutical composition comprising from 0.24 mg to 21.1 mg of diazoxide or a pharmaceutically acceptable salt thereof for use in the treatment of a human afflicted with amyotrophic lateral sclerosis (ALS).

In yet another aspect, the invention relates to a method of treatment of a mammal suffering from amyotrophic lateral sclerosis (ALS), comprising the administration of an amount of diazoxide or a pharmaceutically acceptable salt thereof ranging from 0.15 mg/m$^2$/day to 13.00 mg/m$^2$/day.

## BRIEF DESCRIPTION OF THE FIGURES

**Figure 1.** Graphics showing the blood glucose level (expressed on mg/dl) 60 minutes after diazoxide administration on mice during one-month treatment. **A**: diazoxide dose 1 mg/kg/day (3 mg/m$^2$/day); **B**: diazoxide dose 0.05 mg/kg/day (0.15 mg/m$^2$/day). Results expressed as mean $\pm$ standard error of the mean (SEM).

**Figure 2.** Graphic representation showing the percentage of SOD 1 mutated mice alive at age of 21 weeks after treatment with vehicle and with diazoxide 1 mg/kg/day (3 mg/m$^2$/day), 0.1 mg/kg/day (0.3 mg/m$^2$/day) or 0.05 mg/kg/day (0.15 mg/m$^2$/day).

## DETAILED DESCRIPTION OF THE INVENTION

Inventors have surprisingly found that diazoxide is effective in the treatment of amyotrophic lateral sclerosis (ALS) in a daily dose which is well below the dose used for the treatment of other conditions such as hypoglycemia in humans, thus avoiding the hyperglycaemic effect, which is undesirable when treating a patient with a neurodegenerative disease who normally will not suffered from hypoglycemia. This is especially surprising as the prior art suggest that substantially higher doses of diazoxide are necessary to achieve therapeutic effects in the CNS.

It is one aspect of the present invention the diazoxide or a pharmaceutically acceptable salt thereof for use as a medicament at a daily dose of from 0.15 mg/m$^2$/day to 13.00 mg/m$^2$/day, preferably 0.15 mg/m$^2$/day to 3.70 mg/m$^2$/day, preferably 0.15 mg/m$^2$/day to 3.00 mg/m$^2$/day, preferably 0.15 mg/m$^2$/day to 2.60 mg/m$^2$/day, more preferably 0.15 mg/m$^2$/day to 2.40 mg/m$^2$/day, even more preferably 0.15 5 mg/r$^n$/day to 1.50 mg/m$^2$/day, and most preferably 0.15 mg/m$^2$/day to 0.75 mg/m$^2$/day in the treatment of a mammal afflicted with amyotrophic lateral sclerosis (ALS). It is further preferred that the medicament is prepared for the administration of a daily dose of diazoxide of from 0.15 mg/m$^2$/day to 0.37 mg/m$^2$/day. The quantities are expressed as amount of diazoxide free base.

In a specific embodiment the medicament is prepared for the administration of a daily dose of diazoxide selected from 0.15 mg/m$^2$/day, 1.85 mg/m$^2$/day, 2.22 mg/m$^2$/day, 2.96 mg/m$^2$/day and 11.1 mg/m$^2$/day.

The term "pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

The term "pharmaceutically acceptable salt" embraces salts with a pharmaceutically acceptable acid or base. Pharmaceutically acceptable acids include both inorganic acids, for example and without limitation hydrochloric, sulfuric, phosphoric, diphosphoric, hydrobromic, hydroiodic and nitric acid and organic acids, for example and without limitation citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example and without limitation alkyl amines, arylalkyl amines and heterocyclic amines.

The term "medicament" refers to a pharmaceutical composition comprising diazoxide or a pharmaceutically acceptable salt thereof. The medicament may be administered orally, by injection, by inhalation or insuflation or by the rectal route. All these pharmaceutical compositions are prepared by conventional means with pharmaceutically acceptable excipients. Oral and parenteral formulations are preferred.

In one embodiment of the present invention the medicament is prepared for oral administration. Pharmaceutical compositions for oral administration are in any suitable dosage form such as syrups, solutions, suspensions, tablets, capsules, lozenges, controlled-release preparations, fast-dissolving preparations or they may be presented as a dry product for reconstitution with water or other suitable vehicle before use.

Pharmaceutical compositions for injection (subcutaneous, intradermic, intramuscular, intravenous, etc.) including suspensions, solutions, emulsions in oily or aqueous vehicles, pastes and implantable sustained-release or biodegradable formulations. Such formulations can further comprise one or more additional ingredients including suspending, stabilizing and/or dispersing agents. In one embodiment of a formulation for parenteral administration, the active ingredient is provided in dry (i. e. powder or granular) form for reconstitution with a suitable vehicle (e. g. sterile pyrogen-free water) prior to parenteral administration of the reconstituted composition. Diazoxide may be formulated for parenteral administration by bolus injection or continuous infusion.

Other parentally-administrable formulations which are useful include those which comprise the active ingredient in microcrystalline form, in a liposomal preparation, or as a component of a biodegradable polymer system. Compositions for sustained release or implantation can comprise pharmaceutically acceptable polymeric or hydrophobic materials such as an emulsion, an ion exchange resin, a sparingly soluble polymer, or a sparingly soluble salt.

The pharmaceutical formulations may conveniently be presented in unit dosage form and may be prepared by any of the methods well known in the art of pharmacy. All methods include the step of bringing the active ingredient(s) association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A syrup formulation will generally consist on a suspension or solution of the compound or salt in a liquid carrier for example natural, synthetic or semisynthetic oils or water with flavouring, sweetener and/or colouring agent.

When the composition is in the form of a tablet, any pharmaceutical carrier routinely used for preparing solid formulations may be used.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i. e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered.

Formulations for injection may be presented in unit dosage form (e.g. in ampoules) or in multidose containers with an added preservative.

The term treatment is used to designate the administration of diazoxide or its pharmaceutical acceptable salt or compositions thereof to control disease progression before or after the clinical signs had appeared. By control of the disease progression it is meant to designate beneficial or desired clinical results including, but not limited to, reduction of symptoms, reduction of the length of the disease, stabilization pathological state (specifically avoidance of further deterioration), delay in the disease's progression, improvement of the pathological state and remission (both partial and total). "Control of disease progression" can also entail prolonged survival, compared to the expected survival if the treatment is not applied. In a particular embodiment of the invention diazoxide is used to control of the disease progression once at least one of the disease's clinical signs has appeared.

The term "mammal" includes, but is not restricted to, domestic and farm mammals, primates and humans, e.g., human beings, non-human primates, cows, horses, pigs, sheep, goats, dogs, cats or rodents. In a preferred embodiment the mammal is a human.

Amyotrophic lateral sclerosis (ALS) is a progressive, fatal, neurodegenerative disease caused by the degeneration of motor neurons, the nerve cells in the central nervous system that control voluntary muscle movement. Sporadic and familial ALS (SALS and FALS, respectively) are known. Both are clinically and pathologically similar, suggesting a common pathogenesis.

In the context of the present invention the term "amyotrophic lateral sclerosis (ALS)" includes the spectrum of neurodegenerative syndromes known under the names of Classical (Charcot's) ALS, Lou Gehrig's disease, motor neuron disease (MND), progressive bulbar palsy (PBP), progressive muscular atrophy (PMA), primary lateral sclerosis (PLS), bulbar onset ALS, spinal onset ALS and ALS with multi-system involvement (Wijesekera LC and Leigh PN. Amyotrophic lateral sclerosis. Orphanet Journal of Rare Disease 2009, 4:3).

The transgenic SOD1G93A mouse is an accepted animal model of ALS characterized by having a mutation G93A in the SOD1 gene (Ludolph AC et al. Guidelines for the preclinical in vivo evaluation of pharmacological active drugs for ALS/MND: Report on the 142nd ENMC international workshop. 2007, Amyothr Lat Sclerosis 8:217-223). In said mutant mouse aggregates of mutant SOD1 were found only in diseased tissues, and greater amounts were detected during motor neuron degeneration.

The life expectancy of the commercial available form of the transgenic SOD1G93A mouse differs by between 140 and 160 days and frank paresis appears 10-15 days prior to death.

To date, the ALS-SOD1 mice remain the best model of the disease for preclinical studies. Therefore, the experiments of the present invention were performed in the transgenic SOD1G93A mouse model.

In an embodiment of the present invention the medicament comprises diazoxide as a sole therapeutic agent. However, it may be advantageous to treat ALS with a multiple approach using more than one therapeutic agent useful in the treatment of amyotrophic lateral sclerosis. The beneficial use of diazoxide in the treatment of amyotrophic lateral sclerosis in mammals could also be of high value when combined with another treatment for this disease or for its symptoms. Thus, in another embodiment of the present invention the medicament is prepared for the combined administration of diazoxide and one or more therapeutic agents useful in the treatment of amyotrophic lateral sclerosis.

The term "therapeutic agent useful in the treatment of amyotrophic lateral sclerosis" is referred to an agent suitable to be used to treat amyotrophic lateral sclerosis. Therapeutic agents useful in the treatment of amyotrophic lateral sclerosis include, without limitation, CK-2017357, olesoxime (TRO19622), arimoclomol, riluzole, tretionin and pioglitazone HC1, AVP-923, memantine, talampanel, tauroursodeoxycholic acid (TUDCA), thalidomide, olanzapine, KNS-760704, lithium carbonate, NP001, ONO-2506PO, tamoxifen, creatine monohydrate, coenzyme Q10, YAM80, sodium phenylbutyrate, pyrimethamine, R(+)pramipexole dihydrochloride monohydrate, vitamin E, minocycline, topiramate, gabapentin, AEOL-10150, stem cell injections, SB-509, autologous bone marrow-derived stem cells, ceftriaxone, E0302 (mecobalamin), MCI-186, glatiramer acetate, insulin-like growth factor-1 (IGF-I), ISIS 333611, sNN0029, GSK1223249, brain-derived neurotrophic factor (BDNF), anti-CD40L antibody.

By "combined administration" it has to be understood that diazoxide can be administered together or separately, simultaneously, concurrently or sequentially with a therapeutic agent useful in the treatment of amyotrophic lateral sclerosis in any order, e.g. the administration of diazoxide can be made first, followed by the administration of one or more therapeutic agent(s) useful in the treatment of amyotrophic lateral sclerosis; or the administration of diazoxide can be made last, preceded by the administration of one or more therapeutic agent(s) useful in the treatment of amyotrophic lateral sclerosis; or the administration of diazoxide can be made concomitantly with one or more therapeutic agent(s) useful in the treatment of amyotrophic lateral sclerosis.

A person skilled in the art understands, in the context of the present invention, that the medicament for combined administration of diazoxide and an additional therapeutic agent useful in the treatment of amyotrophic lateral sclerosis can be in the form of a single dosage form or in separate dosage forms. In an embodiment of the present invention, the medicament comprises diazoxide and one or more additional therapeutic agent(s) useful in the treatment of amyotrophic lateral sclerosis selected from CK-2017357, olesoxime (TRO19622), arimoclomol, riluzole, tretionin and pioglitazone HC1, AVP-923, memantine, talampanel, tauroursodeoxycholic acid (TUDCA), thalidomide, olanzapine, KNS-760704, lithium carbonate, NP001, ONO-2506PO, tamoxifen, creatine monohydrate, coenzyme Q10, YAM80, sodium phenylbutyrate, pyrimethamine, R(+)pramipexole dihydrochloride monohydrate, vitamin E, minocycline, topiramate, gabapentin, AEOL-10150, stem cell injections, SB-509, autologous bone marrow-derived stem cells, ceftriaxone, E0302 (mecobalamin), MCI-186, glatiramer acetate, insulin-like growth factor-1 (IGF-I), ISIS 333611, sNN0029, GSK1223249, brain-derived neurotrophic factor (BDNF) and anti-CD40L antibody; preferably riluzole, arimoclomol, anti-CD40L antibody, AVP-923, lithium carbonate, ceftriaxone, NP001, ONO-2506PO, KNS-760704, stem cell injections and GSK1223249.

In another embodiment of the present invention diazoxide and the additional therapeutic agent are contained in a single dosage form. Additional therapeutic agents suitable for combination with diazoxide in a single dosage form are CK-2017357, olesoxime (TRO19622), arimoclomol, riluzole, tretionin and pioglitazone HCI, AVP-923, memantine, talampanel, tauroursodeoxycholic acid (TUDCA), thalidomide, olanzapine, KNS-760704, lithium carbonate, NP001, ONO-2506PO, tamoxifen, creatine monohydrate, coenzyme Q10, YAM80, sodium phenylbutyrate, pyrimethamine, R(+)pramipexole dihydrochloride monohydrate, vitamin E, minocycline, topiramate, gabapentin; preferably riluzole, arimoclomol, AVP-923, KNS-760704, lithium carbonate, NP001 and ONO-2506PO.

In another embodiment of the present invention diazoxide and the additional therapeutic agent are contained in separate dosage forms. Additional therapeutic agents suitable for combined treatment with diazoxide in a separate dosage forms are AEOL-10150, stem cell injections, SB-509, autologous bone marrow-derived stem cells, ceftriaxone, E0302 (mecobalamin), MCI-186, glatiramer acetate, insulin-like growth factor-1 (IGF-I), ISIS 333611, sNN0029, GSK1223249, brain-derived neurotrophic factor (BDNF), anti-CD40L antibody; preferably anti-CD40L antibody, ceftriaxone, stem cell injections and GSK1223249.

According to the present invention, diazoxide is found to be effective for the treatment of ALS at a dose below 13 mg/m$^2$/day (0.351 mg/kg/day in humans) which is much lower than the doses currently used for hypoglycemia and hypertensive treatments (3-8 mg/kg/day and 600-800 mg/day, respectively).

When diazoxide is used for the treatment of a human patient it will be administered in quantities ranging from 0.004 mg/kg/day to 0.351 mg/kg/day, preferably 0.004 mg/kg/day to 0,1 mg/kg/day, preferably 0.004 mg/kg/day to 0.081 mg/kg/day, preferably 0.004 mg/kg/day to 0.07 mg/kg/day, more preferably 0.004 mg/kg/day to 0.065 mg/kg/day, even more preferably 0.004 mg/kg/day to 0.041 mg/kg/day, most preferably 0.004 mg/kg/day to 0.020 mg/kg/day and still most preferably from 0.004 mg/kg/day to 0.010 mg/kg/day. These doses, in the case of a patient having an average body weight of 60 Kg, correspond to daily doses ranging from 0.24 mg/day to 21.1 mg/day, preferably 0.24 mg/day to 6 mg/day, preferably 0.24 mg/day to 4.86 mg/day, preferably 0.24 mg/day to 4.22 mg/day, more preferably 0.24 mg/day to 3.89 mg/day, even more preferably 0.24 mg/day to 2.43 mg/day, most preferably 0.24 mg/day to 1.22 mg/day and still most preferably from 0.24 mg/day to 0.60 mg/day. The quantities are expressed as amount of diazoxide free base.

These daily doses can be administered once a day or divided into two or three equal doses administered along the day. The diazoxide content in the pharmaceutical composition will vary according to the number of daily administrations.

Thus according to another aspect the present invention provides a unit dose composition formulated for administration three times a day will contain from 0.08 mg to 7.03 mg, preferably 0.08 mg to 2 mg, preferably 0.08 mg to 1.62 mg, preferably 0.08 mg to 1.41 mg, more preferably 0.08 mg to 1.30 mg, even more preferably 0.08 mg to 0.81 mg, most preferably 0.08 mg to 0.41 mg and still most preferably from 0.08 mg to 0.20 mg of diazoxide or a pharmaceutically acceptable salt thereof for use in the treatment of a human afflicted with amyotrophic lateral sclerosis (ALS).

According to another aspect the present invention provides a unit dose composition formulated for administration twice a day will contain from 0.12 mg to 10.55 mg, preferably 0.12 mg to 3 mg, preferably 0.12 mg to 2.43 mg, preferably 0.12 mg to 2.11 mg, more preferably 0.12 mg to 1.95 mg, even more preferably 0.12 mg to 1.21 mg, most preferably 0.12 mg to 0.61 mg and still most preferably from 0.12 mg to 0.30 mg of diazoxide or a pharmaceutically acceptable salt thereof for use in the treatment of a human afflicted with amyotrophic lateral sclerosis (ALS).

According to still another aspect the present invention provides a unit dose composition formulated for administration once a day will contain from 0.24 mg to 21.1 mg, preferably 0.24 mg to 6 mg, preferably 0.24 mg to 4.86 mg, preferably 0.24 mg to 4.22 mg, more preferably 0.24 mg to 3.89 mg, even more preferably 0.24 mg to 2.43 mg, most preferably 0.24 mg to 1.22 mg and still most preferably from 0.24 mg to 0.60 mg of diazoxide or a pharmaceutically acceptable salt thereof for use in the treatment of a human afflicted with amyotrophic lateral sclerosis (ALS).

In a preferred embodiment diazoxide is used for the treatment of a human patient in a quantity selected from 0.06 mg/kg/day, 0.08 mg/kg/day and 0.3 mg/kg/day; preferably 0.06 mg/kg/day and 0.08 mg/kg/day; most preferably 0.06 mg/kg/day. These doses, in the case of a patient having an average body weight of 60 Kg, correspond to daily doses of 3.6 mg, 4.8 and 18 mg, respectively. The quantities are expressed as amount of diazoxide free base.

In a particular embodiment the present invention provides a unit dose composition comprising diazoxide or a pharmaceutically acceptable salt thereof in an amount selected from 1.2 mg, 1.6 mg, 1.8 mg, 2.4 mg, 3.6 mg, 4.8 mg, 6 mg, 9 mg and 18 mg; preferably 6 mg, 9 mg and 18 mg; more preferably 1.6 mg, 2.4 mg and 4.8 mg; most preferably 1.2 mg, 1.8 mg and 3.6 mg for use in the treatment of a human afflicted with amyotrophic lateral sclerosis (ALS).

In the present invention, the term "pharmaceutical composition" is equivalent to the term "medicament" as used herein.

The pharmaceutical composition can be an oral dosage form intended for once a day or twice a day administration. This facilitates adhesion of the patient to the therapeutic regime and thus compliance with this regime.

In another aspect, the invention is directed to a method of treatment of a mammal suffering from amyotrophic lateral sclerosis (ALS), comprising the administration of an amount of diazoxide or a pharmaceutically acceptable salt thereof ranging from 0.15 mg/m$^2$/day to 13.00 mg/m$^2$/day, preferably 0.15 mg/m$^2$/day to 3.70 mg/m$^2$/day, preferably 0.15 mg/m$^2$/day to 3.00 mg/m$^2$/day, preferably 0.15 mg/m$^2$/day to 2.60 mg/m$^2$/day, more preferably 0.15 mg/m$^2$/day to 2.40 mg/m$^2$/day, more preferably 0.15 mg/m$^2$/day to 1.90 mg/m$^2$/day, even more preferably 0.15 mg/m$^2$/day to 1.50 mg/m$^2$/day, and most preferably 0.15 mg/m$^2$/day to 0.75 mg/m$^2$/day. It is further preferred to administer an amount of diazoxide or a pharmaceutically acceptable salt thereof ranging from 0.15 mg/m$^2$/day to 0.37 mg/m$^2$/day.

When diazoxide is used for the treatment of a human patient the method of treatment comprises the administration of an amount of diazoxide or a pharmaceutically acceptable salt thereof ranging from 0.004 mg/kg/day to 0.351 mg/kg/day, preferably 0.004 mg/kg/day to 0,1 mg/kg/day, preferably 0.004 mg/kg/day to 0.081 mg/kg/day, preferably 0.004 mg/kg/day to 0.07 mg/kg/day, more preferably 0.004 mg/kg/day to 0.065 mg/kg/day, even more preferably 0.004 mg/kg/day to 0.041 mg/kg/day, most preferably 0.004 mg/kg/day to 0.020 mg/kg/day and still most preferably from 0.004 mg/kg/day to 0.010 mg/kg/day. These doses, in the case of a patient having an average body weight of 60 Kg, correspond to daily doses ranging from 0.24 mg/day to 21.1 mg/day, preferably 0.24 mg/day to 6 mg/day, preferably 0.24 mg/day to 4.86 mg/day, preferably 0.24 mg/day to 4.22 mg/day, more preferably 0.24 mg/day to 3.89 mg/day, even more preferably 0.24 mg/day to 2.43 mg/day, most preferably 0.24 mg/day to 1.22 mg/day and still most preferably from 0.24 mg/day to 0.60 mg/day. The quantities are expressed as amount of diazoxide free base.

In a preferred embodiment diazoxide is used for the treatment of a human patient wherein the method of treatment comprises the administration of an amount of diazoxide or a pharmaceutically acceptable salt thereof selected from 0.06 mg/kg/day, 0.08 mg/kg/day and 0.3 mg/kg/day; preferably 0.06 mg/kg/day and 0.08 mg/kg/day; most preferably 0.06 mg/kg/day. These doses, in the case of a patient having an average body weight of 60 Kg, correspond to daily doses of 3.6 mg, 4.8 and 18 mg, respectively. The quantities are expressed as amount of diazoxide free base.

These daily doses can be administered once a day or divided into two or three equal doses administered along the day. The amount of diazoxide administered will vary according to the number of daily administrations.

Thus according to another aspect the present invention provides a method of treatment comprising the administration three times a day of an amount of diazoxide or a pharmaceutically acceptable salt thereof ranging from 0.08 mg to 7.03 mg, preferably 0.08 mg to 2 mg, preferably 0.08 mg to 1.62 mg, preferably 0.08 mg to 1.41 mg, more preferably 0.08 mg to 1.30 mg, even more preferably 0.08 mg to 0.81 mg, most preferably 0.08 mg to 0.41 mg and still most preferably from 0.08 mg to 0.20 mg.

In yet another aspect the present invention provides a pharmaceutical composition comprising from 0.08 mg to 7.03 mg, preferably 0.08 mg to 2 mg, preferably 0.08 mg to 1.62 mg, preferably 0.08 mg to 1.41 mg, more preferably 0.08 mg to 1.30 mg, even more preferably 0.08 mg to 0.81 mg, most preferably 0.08 mg to 0.41 mg and still most preferably from 0.08 mg to 0.20 mg of diazoxide or a pharmaceutically acceptable salt thereof for administration three times a day in the treatment of amyotrophic lateral sclerosis (ALS).

According to another aspect the present invention provides a method of treatment comprising the administration twice a day of an amount of diazoxide or a pharmaceutically acceptable salt thereof ranging from 0.12 mg to 10.55 mg, preferably 0.12 mg to 3 mg, preferably 0.12 mg to 2.43 mg, preferably 0.12 mg to 2.11 mg, more preferably 0.12 mg to 1.95 mg, even more preferably 0.12 mg to 1.21 mg, most preferably 0.12 mg to 0.61 mg and still most preferably from 0.12 mg to 0.30 mg.

In yet another aspect the present invention provides a pharmaceutical composition comprising from 0.12 mg to 10.55 mg, preferably 0.12 mg to 3 mg, preferably 0.12 mg to 2.43 mg, preferably 0.12 mg to 2.11 mg, more preferably 0.12 mg to 1.95 mg, even more preferably 0.12 mg to 1.21 mg, most preferably 0.12 mg to 0.61 mg and still most preferably from 0.12 mg to 0.30 mg of diazoxide or a pharmaceutically acceptable salt thereof for administration twice a day in the treatment of amyotrophic lateral sclerosis (ALS).

According to still another aspect the present invention provides a method of treatment comprising the administration once a day of an amount of diazoxide or a pharmaceutically acceptable salt thereof ranging from 0.24 mg to 21.1 mg, preferably 0.24 mg to 6 mg, preferably 0.24 mg to 4.86 mg, preferably 0.24 mg to 4.22 mg, more preferably 0.24 mg to 3.89 mg, even more preferably 0.24 mg to 2.43 mg, most preferably 0.24 mg to 1.22 mg and still most preferably from 0.24 mg to 0.60 mg.

In yet another aspect the present invention provides a pharmaceutical composition comprising from 0.24 mg to 21.1 mg, preferably 0.24 mg to 6 mg, preferably 0.24 mg to 4.86 mg, preferably 0.24 mg to 4.22 mg, more preferably 0.24 mg to 3.89 mg, even more preferably 0.24 mg to 2.43 mg, most preferably 0.24 mg to 1.22 mg and still most preferably from 0.24 mg to 0.60 mg of diazoxide or a pharmaceutically acceptable salt thereof for administration once a day in the treatment of amyotrophic lateral sclerosis (ALS).

In yet another aspect the present invention provides a pharmaceutical composition for use in the treatment of a human afflicted with amyotrophic lateral sclerosis (ALS) characterized in that it comprises from 0.24 mg to 21.1 mg, preferably 0.24 mg to 6 mg, preferably 0.24 mg to 4.86 mg, preferably 0.24 mg to 4.22 mg, more preferably 0.24 mg to 3.89 mg, even more preferably 0.24 mg to 2.43 mg, most preferably 0.24 mg to 1.22 mg and still most preferably from 0.24 mg to 0.60 mg of diazoxide or a pharmaceutically acceptable salt thereof.

In a particular embodiment the present invention provides a pharmaceutical composition comprising an amount selected from 1.2 mg, 1.6 mg, 1.8 mg, 2.4 mg, 3.6 mg, 4.8 mg, 6 mg, 9 mg and 18 mg; preferably 6 mg, 9 mg and 18 mg; more preferably 1.6 mg, 2.4 mg and 4.8 mg; most preferably 1.2 mg, 1.8 mg and 3.6 mg of diazoxide or a pharmaceutically acceptable salt thereof for administration in the treatment of amyotrophic lateral sclerosis (ALS).

In a particular embodiment the present invention provides a pharmaceutical composition for use in the treatment of a human afflicted with amyotrophic lateral sclerosis (ALS) characterized in that it comprises an amount of diazoxide or a pharmaceutically acceptable salt thereof selected from 1.2 mg, 1.6 mg, 1.8 mg, 2.4 mg, 3.6 mg, 4.8 mg, 6 mg, 9 mg and 18 mg; preferably 6 mg, 9 mg and 18 mg; more preferably 1.6 mg, 2.4 mg and 4.8 mg; most preferably 1.2 mg, 1.8 mg and 3.6 mg.

In particular embodiments diazoxide or a pharmaceutically acceptable salt thereof is administered in an amount selected from 2.22 mg/m$^2$/day, 2.96 mg/m$^2$/day and 11.1 mg/m$^2$/day.

In one embodiment the method is characterized in that diazoxide or a pharmaceutically acceptable salt thereof is administered orally.

In one embodiment of the present invention the method is characterized in that diazoxide or a pharmaceutically acceptable salt thereof is administered as a sole therapeutic agent. In other embodiment the method comprises the administration of an amount of diazoxide or a pharmaceutically acceptable salt thereof in combination with one or more therapeutic agents useful in the treatment of amyotrophic lateral sclerosis (ALS). In a preferred embodiment the diazoxide and the additional therapeutic agent are contained in a single dosage form. In other preferred embodiment diazoxide and the additional therapeutic agent are contained in separate dosage forms.

In one embodiment of the present invention the method is characterized in that diazoxide or a pharmaceutically acceptable salt thereof is administered as a dosage form intended for once a day administration. In another embodiment it is intended for twice a day administration.

In a preferred embodiment of the present invention the method is used to treat humans.

## EXAMPLES

Experiments in the present invention, performed in mice, had shown that diazoxide is effective at different daily doses below 13 mg/m$^2$/day.

Doses of diazoxide may be expressed either in mg of diazoxide per kg of body weight or in mg of diazoxide per square meter of body surface. The article from Reagan-Shaw S. "Dose translation from animal to human studies revisited". FASEB J 2007, 22:659-661 provides the standard conversion factors used to convert mg/kg to mg/m$^2$.

$$\text{Dose (mg/kg)} \times K_m = \text{Dose (mg/m}^2\text{)}$$

The article also explains that this conversion is the basis for converting dose in a first animal species to dose in a second animal species (allometric dose translation). Thus, animal dose (AD) in mg/kg can be converted to human equivalent dose (HED) in mg/kg using the following formula:

$$\text{HED (mg/kg)} = \text{AD (mg/kg)} \times \frac{\text{Animal } K_m}{\text{Human } K_m}$$

wherein the $K_m$ for each species is shown in Table II (data extracted from Reagan-Shaw S. Dose translation from animal to human studies revisited. FASEB J 2007, 22:659-661).

**Table II. $K_m$ factor for conversion of AD to HED**

| Species | | $K_m$ factor |
|---|---|---|
| Human | Adult | 37 |
| | Child | 25 |
| | Baboon | 20 |
| | Dog | 20 |
| | Monkey | 12 |
| | Rabbit | 12 |
| | Guinea pig | 8 |
| | Rat | 6 |
| | Hamster | 5 |
| | Mouse | 3 |

Thus, the experiments with doses of 0.05 mg/kg/day, 0.1 mg/kg/day, 0.8 mg/kg/day, 1 mg/kg/day and 4 mg/kg/day in mice correspond to general doses in mammals of 0.15 mg/m$^2$/day, 0.3 mg/m$^2$/day, 2.4 mg/m$^2$/day, 3 mg/m$^2$/day and 12 mg/m$^2$/day.

The advantages of the invention are more fully illustrated with reference to the following examples.

### EXAMPLE 1: LOW DOSES OF DIAZOXIDE DO NOT CAUSE HYPERGLYCEMIA IN MICE

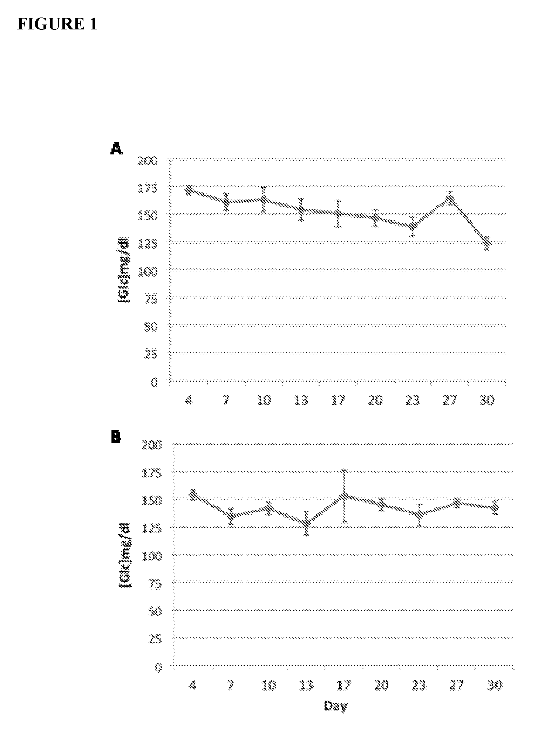

To determine the *in vivo* effects of very low doses of diazoxide on glycemia, mice receiving a daily administration of diazoxide were monitorized. Female C57BL/6J mice, 11 weeks of age, were purchased from Charles River and maintained on a 12:12 hr light:dark cycle, with standard chow and water freely available. The experiments began at 11:00 h. Diazoxide (Sigma-Aldrich, St. Louis, Mo, USA) was administered daily orally by gavage (p.o.) at doses 1 mg/kg (3 mg/m$^2$) (Figure 1A) and 0.05 mg/kg (0.15 mg/m$^2$) (Figure 1B) (n = 6/group) for an initial period of 4 days. This period of time was considered necessary to create a steady state in plasma diazoxide concentration. From day 4 on, glucose blood levels were measured every 3-4 days during the 30-day treatment period. Measurements were performed immediately before drug administration (time 0) and at 60 minutes. Blood samples were obtained from the saphenous vein and glucose levels were determined using a glucometer and glucose test strips (Accu-Chek® Aviva, Roche Diagnostics, Indianapolis, Ind, USA). For each group, hyperglycemia was considered when glucose concentration was equal or higher than 176 mg/dl. As shown in FIGURE 1 none of the two doses tested in this study produced hyperglycemia after a 30 days period of diazoxide treatment.

### EXAMPLE 2: LOW DOSES OF DIAZOXIDE IMPROVE SURVIVAL ON C57BL/6J-TgN(SOD1-G93A)1Gur[dl] TRANSGENIC MICE MODEL FOR AMYOTROPHIC LATERAL SCLEROSIS

Transgenic C57BL/6J-TgN(SOD1-G93A)1Gur[dl] mice were originally purchased from The Jackson Laboratories (Bar Harbor, ME) and routinely identified as positive carriers of the transgene at early postnatal age by PCR amplification (Garney et al. Motor neuron degeneration in mice that express a human Cu,Zn superoxide dismutase mutation. Science 1994. 264(5166):1772-5). The animal colony was kept under controlled temperature (22$\pm$2$^0$C), humidity (40-60%) and light (12 h cycles), and treated in accordance with the European Community Council directive on animal welfare (86/609/EEC).

At 70 days postnatal age, mice identified as positive carriers of the SOD1-G93A transgene were randomly assigned to a diazoxide-treatment or control group. An additional group composed by wild type C57BL/6J was also used as absolute control for spontaneous deaths. The treatment started at postnatal day 70 and lasted until the animal was sacrificed. The drug was administered daily orally by gavage (p.o.), in a dose volume of 200 $\mu$l. Diazoxide (Sigma-Aldrich, St. Louis, Mo, USA) was administered at doses of 0.05 mg/kg/day (0.15 mg/m$^2$/day), 0.1 mg/kg/day (0.3 mg/m$^2$/day) and 1 mg/kg/day (3 mg/m$^2$/day). One control group received a daily oral administration of the vehicle where diazoxide was dissolved consisting in 98.5% water plus 1.5% of dimethyl sulfoxide (Sigma-Aldrich, St. Louis, Mo, USA). Each group consists of 14 animals.

Around 75 days of postnatal age treated and not treated mice began to display less exploratory activity and less feeding behaviour as well as a loss of body weight. 20-30 days after, motor deficits were clearly observable, with a continuous loss of muscular mass and body weight. Between postnatal day 137 and 172 animals began to not be able to right themselves within 30 seconds from either side being this their last period of survival. Date and cause of death were recorded for each mouse. For humanitarian reasons, animals were closely monitored and sacrificed as moribund prior to actual death using criteria for severe moribund state. To determine duration of survival reliably and humanely, the moribund state, defined as the inability of mice to right themselves 30 seconds after being placed on a side was used. The moribund mice were considered as "dead", and were euthanized using carbon dioxide. It is known that when animals reach this "moribund" stage they are unable to survive more than 24 additional hours.

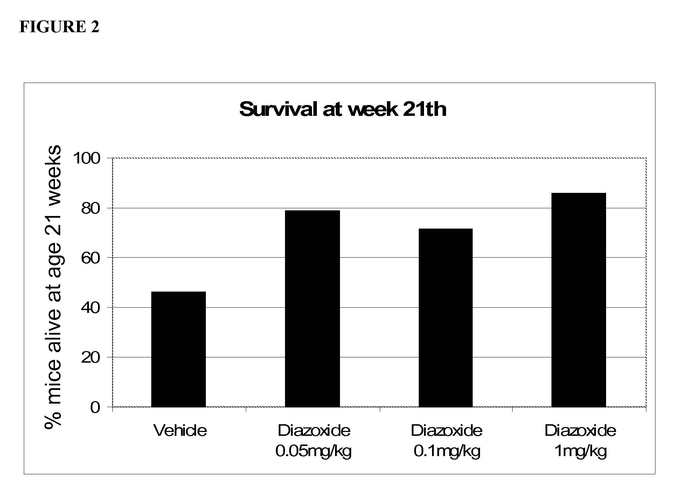

At the 21th postnatal week only 46.2% of C57BL/6J-TgN(SOD1-G93A)1Gur[dl] mice treated with vehicle survived whereas 85.7% of the animals treated with diazoxide 1mg/kg/day (3 mg/m²/day) were still alive. For the dosages of 0.1 mg/kg/day (0.3 mg/m²/day) and 0.05 mg/kg/day (0.15 mg/m²/day), the percentage of survival at this age was of 71.4% and 78.6%, respectively (Figure 2 and Table III). For the wild type - non transgenic control group the survival at this age was 100%.

**Table III. Percentage of mice alive at age of 21 weeks.**

| Treatment | Vehicle | Diazoxide 0.05mg/kg/day (0.15mg/m²/day) | Diazoxide 0.1 mg/kg/day (0.3mg/m²/day) | Diazoxide 1mg/kg/day (3mg/m²/day) |
|---|---|---|---|---|
| **Survival at week 21th** | 46.2 % | 78.6 % | 71.4 % | 85.7% |

All diazoxide dosages tested showed a better median value for survival when compared to the control group. The median survival for vehicle control group was 147 days, for diazoxide 1mg/kg/day (3 mg/m²/day) it was 156 days, for 0.1 mg/kg/day (0.3 mg/m²/day) it was 152 days and for the dose 0.05 mg/kg/day (0.15 mg/m²/day) it was 153 days (Table IV).

**Table IV. Median survival for vehicle and diazoxide treated mice.**

| Treatment | Vehicle | Diazoxide 0.05mg/kg/day (0.15mg/m²/day) | Diazoxide 0.1mg/kg/day (0.3mg/m²/day) | Diazoxide 1mg/kg/day (3mg/m²/day) |
|---|---|---|---|---|
| **Median Survival (days)** | 147 | 153 | 152 | 156 |

**Claims**

1. Diazoxide or a pharmaceutically acceptable salt thereof for use as a medicament at a daily dose of from 0.15 mg/m²/day to 13.00 mg/m²/day expressed as mg/m²/day of diazoxide free base in the treatment of a mammal afflicted with amyotrophic lateral sclerosis (ALS).

2. Diazoxide or a pharmaceutically acceptable salt thereof for use according to claim 1 wherein diazoxide in the form of free base is used.

3. Diazoxide or a pharmaceutically acceptable salt thereof for use according to any preceding claim, wherein the mammal is a human.

4. Diazoxide or a pharmaceutically acceptable salt thereof for use according to any preceding claim, wherein the medicament is prepared for oral administration.

5. Diazoxide or a pharmaceutically acceptable salt thereof for use according to any preceding claim, wherein the medicament is prepared for the administration of a daily dose of diazoxide of from 0.15 mg/m²/day to 3.00 mg/m²/day.

6. Diazoxide or a pharmaceutically acceptable salt thereof for use according to claims 1 to 4, wherein the medicament is prepared for the administration of a daily dose of diazoxide selected from 0.15 mg/m²/day, 2.22 mg/m²/day, 2.96 mg/m²/day and 11.1 mg/m²/day.

7. Diazoxide or a pharmaceutically acceptable salt thereof for use according to any preceding claim, wherein the medicament comprises diazoxide as sole therapeutic agent.

8. Diazoxide or a pharmaceutically acceptable salt thereof for use according to claims 1 to 6, wherein the medicament is prepared for the combined administration of diazoxide and one or more therapeutic agents useful in the treatment of amyotrophic lateral sclerosis.

9. Diazoxide or a pharmaceutically acceptable salt thereof for use according to claim 8, wherein the medicament comprises diazoxide and one or more additional therapeutic agents useful in the treatment of amyotrophic lateral sclerosis selected from CK-2017357, olesoxime (TRO19622), arimoclomol, riluzole, tretionin and pioglitazone HC1, AVP-923, memantine, talampanel, tauroursodeoxycholic acid (TUDCA), thalidomide, olanzapine, KNS-760704, lithium carbonate, NP001, ONO-2506PO, tamoxifen, creatine monohydrate, coenzyme Q10, YAM80, sodium phenylbutyrate, pyrimethamine, R(+)pramipexole dihydrochloride monohydrate, vitamin E, minocycline, topiramate, gabapentin, AEOL-10150, stem cell injections, SB-509, autologous bone marrow-derived stem cells, ceftriaxone, E0302 (mecobalamin), MCI-186, glatiramer acetate, insulin-like growth factor-1 (IGF-I), ISIS 333611, sNN0029, GSK1223249, brain-derived neurotrophic factor (BDNF) and anti-CD40L antibody.

10. Diazoxide or a pharmaceutically acceptable salt thereof for use according to claim 8 wherein diazoxide and the additional therapeutic agent are contained in a single dosage form.

11. Diazoxide or a pharmaceutically acceptable salt thereof for use according to claim 8 wherein diazoxide and the additional therapeutic agent are contained in separate dosage forms.

12. Pharmaceutical composition comprising from 0.24 mg to 21.1 mg of diazoxide or a pharmaceutically acceptable salt thereof for use in the treatment of a human afflicted with amyotrophic lateral sclerosis (ALS).

13. Pharmaceutical composition for use according to claim 12, **characterized in that** it comprises from 0.24 mg to 4.86 mg of diazoxide or a pharmaceutically acceptable salt thereof.

14. Pharmaceutical composition for use according to claim 12, **characterized in that** it comprises an amount of diazoxide or a pharmaceutically acceptable salt thereof selected from 1.2 mg, 1.6 mg, 1.8 mg, 2.4 mg, 3.6 mg, 4.8 mg, 6 mg, 9 mg and 18 mg.

**FIGURE 1**

**FIGURE 2**

Survival at week 21th

Europäisches
Patentamt

European
Patent Office

Office européen
des brevets

## EUROPEAN SEARCH REPORT

**Application Number**

EP 10 38 2233

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 782 812 A1 (NEUROTEC PHARMA S L [ES]) 9 May 2007 (2007-05-09) * paragraphs [0014], [0021] - [0022]; claims 9,17,26 * | 1-8, 12-14 | INV. A61K31/549 A61P25/14 A61P25/28 |
| X | US 2002/091144 A1 (MARBAN EDUARDO [US] ET AL) 11 July 2002 (2002-07-11) * paragraphs [0030], [0 41]; claim 7; examples 1,2 * | 1-8, 12-14 | |
| X | WO 2006/050165 A2 (YOO SEO HONG [US]) 11 May 2006 (2006-05-11) * paragraphs [0062], [0077] - paragraphs [0003], [0004], [0018], [0029], [0052], [0053], [0055], [0057] * | 1-14 | |

**TECHNICAL FIELDS SEARCHED (IPC)**

A61K
A61P

The present search report has been drawn up for all claims

| Place of search | Date of completion of the search | Examiner |
|---|---|---|
| Munich | 12 January 2011 | Ansaldo, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 38 2233

This annex lists the patent family members relating to the patent documents cited in  the above-mentioned European search report.
The members are as contained in the European Patent Office EDP file on
The European Patent Office is in no way liable for these particulars which are merely given for the purpose of information.

12-01-2011

| Patent document cited in search report | | | Publication date | Patent family member(s) | | Publication date |
|---|---|---|---|---|---|---|
| EP 1782812 | A1 | | 09-05-2007 | AU 2005256675 A1 | | 05-01-2006 |
| | | | | CA 2571717 A1 | | 05-01-2006 |
| | | | | WO 2006000607 A1 | | 05-01-2006 |
| | | | | JP 2008503548 T | | 07-02-2008 |
| | | | | US 2007254871 A1 | | 01-11-2007 |
| US 2002091144 | A1 | | 11-07-2002 | NONE | | |
| WO 2006050165 | A2 | | 11-05-2006 | AU 2005302452 A1 | | 11-05-2006 |
| | | | | CA 2585471 A1 | | 11-05-2006 |
| | | | | EP 1814558 A2 | | 08-08-2007 |
| | | | | EP 2255812 A1 | | 01-12-2010 |
| | | | | JP 2008518935 T | | 05-06-2008 |
| | | | | KR 20070089926 A | | 04-09-2007 |

EPO FORM P0459

For more details about this annex : see Official Journal of the European Patent Office, No. 12/82

## REFERENCES CITED IN THE DESCRIPTION

**Patent documents cited in the description**

- WO 2006000607 A1

**Non-patent literature cited in the description**

- **WIJESEKERA LC ; LEIGH PN.** Amyotrophic lateral sclerosis. *Orphanet Journal of Rare Disease,* 2009, vol. 4, 3
- **WORMS PM.** The epidemiology of motor neuron disease: a review of recent studies. *J Neurol Sci,* 2001, vol. 191, 3-9
- **MITCHELL JD ; BORASIO GD.** Amyotrophic lateral sclerosis. *Lancet,* 2007, vol. 369, 2031-41
- **PASINELLI P ; BROWN RH.** Molecular biology of amyotrophic lateral sclerosis: insight from genetics. *Nat Rev Neurosci,* 2006, vol. 7, 710-723
- **COZZOLINO M et al.** Amyotrophic Lateral Sclerosis: From current developments in the laboratory to clinical implications. *Antiox Redox Sign,* 2008, vol. 10, 406-43
- **TURNER MR et al.** Evidence of widespread cerebral microglial activation in amyotrophic lateral sclerosis: an [11C](R)-PK11195 positron emission tomography study. *Neurobiol Dis,* 2004, vol. 15, 601-609
- **GURNEY ME et al.** Motor neuron degeneration in mice that express a human Cu/Zn superoxide dismutase mutation. *Science,* 1994, vol. 264, 1772-5
- **LUDOLPH AC et al.** Guidelines for the preclinical in vivo evaluation of pharmacological active drugs for ALS/MND: Report on the 142nd ENMC international workshop. *Amyothr Lat Sclerosis,* 2007, vol. 8, 217-223

- **KOLLEWE K et al.** Amyotrophic lateral sclerosis: Current clinical trials and underlying pathomechanisms. *Nervenartz,* 2008, vol. 79, 653-61
- **RAMONET D et al.** Putative glucosensing property in rat and human activated microglia. *Neurobiol Dis,* 2004, vol. 17, 1-9
- **MANNHOLD R.** KATP channel openers: structure-activity relationships and therapeutic potential. *Med Res Rev,* 2004, vol. 24, 213-66
- **FANG P ; MACDONALD I ; LAVER M ; HUA A ; KINCAID-SMITH P.** Oral diazoxide in uncontrolled malignant hypertension. *Med J Aust.,* 26 October 1974, vol. 2 (17), 621-4
- **GANTENBEIN M et al.** *Life Sciences,* 1995, vol. 57, 113-116
- **FARKAS E et al.** *Brain Research,* 2004, vol. 1008, 252-260
- **LENZSER G et al.** *Brain Research.,* 2005, vol. 1051, 72-80
- **REAGAN-SHAW S.** Dose translation from animal to human studies revisited. *FASEB J,* 2007, vol. 22, 659-661
- **REAGAN-SHAW S.** Dose translation from animal to human studies revisited. *FASEB J,* 2007, vol. 22, 659-661
- **GARNEY et al.** Motor neuron degeneration in mice that express a human Cu,Zn superoxide dismutase mutation. *Science,* 1994, vol. 264 (5166), 1772-5